# EUROPÄISCHE PATENTANMELDUNG

(11) **EP 2 881 105 A1**
(43) Veröffentlichungstag der Anmeldung: **10.06.2015**
(21) Anmeldenummer: 14195741.5
(22) Anmeldetag: 02.12.2014
(51) Int. Cl.: A61K 8/66, A61K 8/67, A61Q 11/00, A61K 8/02, A61K 8/04, A61K 8/19, A23L 1/00, A23L 1/302, A23L 1/304

(54) **Zahnpflegeprodukt-Set**

(30) Priorität: 05.12.2013 DE 102013113539
(71) Anmelder: PM-International AG, 1618 Luxembourg (LU)
(72) Erfinder: Sorg, Rolf, 5444 Schengen (LU); Kühne, Tobias, Dr., 54497 Morbach (DE); Trappe-Krieger, Ulrike, Dr., 55743 Idar-Oberstein (DE); Lauinger, Christian, 76275 Ettlingen (DE); Messer, Wilhelm, 67098 Bad Dürkheim (DE)
(74) Vertreter: Zech, Stefan Markus

(57) **Zusammenfassung**

Es wird ein Zahnpflegeprodukt-Set vorgeschlagen umfassend zwei sich in ihrer Wirkung ergänzende Präparate,
nämlich ein erstes, oral einzunehmendes Präparat,
- das als Pulver vorliegt und
- das Calcium und/oder Magnesium
- und Vitamin C

umfasst
sowie ein zweites, auf den Zahnapparat bzw. das Zahnfleisch aufzubringendes Präparat,
- das als Gel vorliegt und
- Coenzym Q10
- und Vitamin E, vorzugsweise Tocopherol, insbesondere α-Tocopherol, umfasst.

## Beschreibung

Die Erfindung betrifft ein Zahnpflegeprodukt-Set.

Es sind bereits eine Vielzahl von Zahnpflegeprodukten bekannt, die in unterschiedlichen Erscheinungsformen angeboten werden, wie beispielsweise Zahnpasten, Mundwässer, etc. Dabei geschieht die Zahnpflege in weiten Kreisen der Bevölkerung in erster Linie durch eine Reinigung und Behandlung des Zahnapparates und des Zahnfleisches von außen über das Putzen der Zähne mit entsprechenden Zahnpasten, Behandeln der Mundhöhle mit Mundwässern, etc.

Die vorliegende Erfindung stellt sich die Aufgabe, die Zahnpflege durch ein Zahnpflegeprodukt-Set noch weiter zu unterstützen. Diese Aufgabe wird mit einem Zahnpflegeprodukt-Set nach den Merkmalen des Anspruchs 1 gelöst.

Nach einem allgemeinen Kerngedanken der vorliegenden Erfindung umfasst das Zahnpflegeprodukt (mindestens) zwei Präparate, nämlich ein erstes oral einzunehmendes Präparat sowie ein zweites, direkt auf den Zahnapparat bzw. das Zahnfleisch aufzubringendes Präparat. Insofern zeichnet sich das Zahnpflege-produkt-Set dadurch aus, dass eine Zahnpflege von "außen mit einer Zahnpflege von "innen" kombiniert wird. Ein zentraler Aspekt der Erfindung liegt also darin, (im Unterschied zum Stand der Technik) eine synergistische Kombination der zwei Präparate vorzuschlagen. Dabei wird insbesondere berücksichtigt, dass ein Antransport von Wirkstoffen von außen schon wegen der Durchdringung der Schleimhäute nicht unproblematisch erscheint. Weiterhin können Taschen so tief sein, dass sie von dem vorhandenen Wirkstoff nicht effektiv erreicht werden. Dadurch kann effektiv Parodontitis und/oder Gingivitis entgegengewirkt werden. Insbesondere ist festzustellen, dass es sich bei parodontalen Erkrankungen um eine ausgeprägte Vielfalt an Symptomatiken handelt (bedingt durch die Vielfalt der betroffenen Gewebe und deren Zellen). Aus diesem Grund ist der vorliegende diversifizierte Therapieansatz besonders wirkungsvoll. Die vorgeschlagene systemische Verabreichung in Kombination mit einer lokalen Applikation (also "von innen und außen") ist insgesamt einer einzelnen Verabreichung (entweder von innen oder außen), wie im Stand der Technik beschrieben, weit überlegen. Konkret wird vorgeschlagen, dass das erste oral einzunehmende Präparat als Pulver vorliegt und Calcium und/oder Magnesium und Vitamin C umfasst. Die Gabe von Calcium und/oder Magnesium unterstützt die Erhaltung von Kieferknochen und Zähnen bzw. kann die Collagenbildung für die normale Funktion der Knochen, des Zahnfleisches und der Zähne unterstützen. Calcium und Vitamin C unterstützen sich dabei bei der Aufnahme bzw. bei der Bioverfügbarkeit. Gleichzeitig wirkt Vitamin C (Ascorbinsäure) collagenbildend bzw. collagenerhaltend. Eine ausreichende Gabe an Vitamin C verhindert Skorbuterscheinungen. Insofern erfüllt Vitamin C beim vorliegenden Nahrungsergänzungspräparat eine Doppelfunktion und wirkt einerseits selbst collagenbildend bzw. collagenunterstützend bzw. skorbutverhindernd und begünstigt gleichzeitig die Aufnahme von Calcium und/oder Magnesium, was unmittelbar dem Zahnaufbau bzw. dem Kieferknochenaufbau zugutekommt. Calcium ist weiterhin bedeutsam zum Aufbau von Wurzelzement oder dem Alveolarknochen bzw. zum Aufbau von Hydroxylapatit.

Das zweite, auf den Zahnapparat bzw. das Zahnfleisch aufzubringende Präparat liegt als Gel vor und umfasst Coenzym Q10 (Ubichinon-10) und Vitamin E. Coenzym Q10 und Vitamin E, vorzugsweise Tocopherol, ergänzen sich in synergistischer Weise.

In einer möglichen, bevorzugten Ausgestaltung liegt das erste Präparat als wasserlösliches Pulver vor, wobei das Nahrungsergänzungspräparat als Getränk eingenommen wird. Durch die Lösung in Wasser wird das Nahrungsergänzungspräparat am Körper besser aufgenommen. Es wird insgesamt eine höhere Bioverfügbarkeit erreicht. Das erste Präparat kann auch als Getränk vorliegen.

In einer bevorzugten Ausgestaltung umfasst das erste Präparat weiterhin Inulin und/oder Citronensäure.

In einer möglichen Ausgestaltung kann im Hinblick auf das erste Präparat vorgesehen sein, dass das Calcium als Calciumcarbonat und/oder als Calciumlactat vorliegt und/oder das Magnesium als Magnesiumcarbonat und/oder Magnesiumcitrat, insbesondere als Trimagnesiumcitrat vorliegt. Calcium, insbesondere Calciumcarbonat, vor allem Calciumlactat unterstützt die Remineralisierung von Zahnschmelz.

In einer bevorzugten Ausgestaltung des ersten Präparats des Zahnpflegeprodukt-Sets ist vorgesehen, dass bezogen auf eine Pulvermenge von 100 g die Menge an Calcium, das insbesondere als Calciumcarbonat und/oder als Calciumlactat vorliegt, zwischen 10 und 25 g, vorzugsweise zwischen 12 und 18 g beträgt und/oder die Menge an Magnesium, das insbesondere als Magnesiumcarbonat und/oder als Magnesiumcitrat vorliegt, zwischen 5 g und 20 g, vorzugsweise zwischen 8 g und 15 g beträgt und/oder die Menge an Vitamin C zwischen 0,2 g und 1 g, vorzugsweise zwischen 0,4 g und 0,6 g liegt und/oder die Menge an Inulin zwischen 30 und 50 g, vorzugsweise zwischen 40 und 45 g beträgt und/oder die Menge an Zitronensäure zwischen 15 g und 30 g, vorzugsweise zwischen 20 g und 28 g beträgt.

In einer weiterhin konkret bevorzugten Ausgestaltung des ersten Präparats des Zahnpflegeprodukt-Sets kann vorgesehen sein, dass bezogen auf eine Pulvermenge von 100 g Calciumcarbonat in einer Konzentration von 10 g bis 20 g, vorzugsweise von 12 bis 14 g und/oder Calciumlactat in einer Konzentration von 2 bis 5 g, vorzugsweise 2,5 bis 4 g und/oder Magnesiumcarbonat (Magnesiumcarbonat x₅H₂O) in einer Konzentration von 5 g bis 20 g, vorzugsweise von 8 g bis 13 g und/oder Magnesiumcitrat (bzw. Trimagnesiumcitrat) in einer Konzentration von 0,7 bis 2,5 g, vorzugsweise von 1 g bis 2 g, enthalten sind.

Es kann weiterhin vorgesehen sein, dass das erste Präparat des Zahnpflege-produkt-Sets ergänzend noch weitere Bestandteile, insbesondere Algenpulver, vorzugsweise Braunalgenpulver, und/oder ein Algenextrakt, insbesondere Braunalgenextrakt, und/oder Betacarotin und/oder Geschmacks- und/oder Aromastoffe und/oder Süßstoffe umfasst. Weiterhin kann das erste Präparat des Zahnpflege-Produkt-Sets Zink und/oder Selen und/oder Q10 umfassen. Vitamin C, Betacarotin, Zink, Selen und Q10 haben eine grundsätzlich antientzündliche Wirkung. Insbesondere Vitamin C, Q10 und Zink sorgen für ein elastisches und collagenes Bindegewebe (insbesondere der Sharpey-Fasern und der Wurzelhaut). Weiterhin kann eine Steviakomponente vorgesehen sein, beispielsweise in Pulverform oder als Extrakt.

In einer konkret möglichen Ausgestaltung kann vorgesehen sein, dass bezogen auf eine Pulvermenge von 100 g Algenpulver in einer Konzentration von 0,1 g bis 0,8 g und/oder Betacarotin in einer Konzentration von 5 mg bis 35 mg, vorzugsweise zwischen 10 mg und 20 mg und/oder Geschmacks- und/oder Aromastoffe in einer Konzentration von 1 g bis 4 g, insbesondere in einer Konzentration von 2 g bis 3 g und/oder Süßstoffe, vorzugsweise in Form eines Steviosids (E960) in einer Konzentration von 0,2 g bis 1,2 g, weiter vorzugsweise in einer Konzentration von 0,5 g bis 0,9 g enthalten sind.

In einer bevorzugten Ausgestaltung des Zahnpflegeprodukt-Sets nach der vorliegenden Erfindung kann vorgesehen sein, dass das im zweiten Präparat vorgesehene Coenzym Q10 (Ubichinon-10) in mycellierter Form vorliegt.
Hierdurch ist es möglich, mit dem Zahnpflegeprodukt eine größere Menge von Q10 stabil und homogen verteilt an den Wirkort zu bringen und somit eine höhere Bioverfügbarkeit zu gewährleisten.

In einer bevorzugten Ausgestaltung kann vorgesehen sein, dass beim zweiten Präparat pro ml Gel Coenzym Q10 in einer Konzentration von 5 mg bis 18 mg, vorzugsweise von 6 mg bis 15 mg, insbesondere bevorzugt von 11 mg bis 13 mg enthalten ist.

In einer weiterhin bevorzugten Ausgestaltung ist beim zweiten Präparat bezogen auf 1 mg Vitamin E (vorzugsweise Tocopherol, insbesondere α-Tocopherol) in einer Konzentration von 8 mg bis 25 mg, vorzugsweise von 10 mg bis 20 mg, besonders bevorzugt von 12 mg bis 17 mg enthalten.

Das zweite Präparat des erfindungsgemäßen Zahnpflegeprodukt-Sets kann weiterhin Krausminze, vorzugsweise in einer Konzentration bezogen auf das Gel von 1 Gew.-% bis 4 Gew.-%, weiter vorzugsweise in einer Konzentration von 2 Gew.-% bis 3 Gew.-% umfassen.

Ferner wird es bevorzugt, wenn zumindest das zweite Präparat weiterhin Konservierungsstoffe, insbesondere Sorbinsäure und/oder eine Sorbatmischung umfasst.

Beim Zahnpflegeprodukt-Set nach der vorliegenden Erfindung kann das in Gelform vorliegende zweite Präparat in einer Flasche mit einem Füllvolumen von 20 ml bis 40 ml, vorzugsweise von etwa 30 ml, konfektioniert sein.

Die Tagesdosis des ersten Präparats des Zahnpflegeprodukt-Sets, das als Pulver vorliegt, kann zwischen 3 g und 15 g, vorzugsweise zwischen 5 g und 8 g betragen, wobei weiterhin vorzugsweise diese Tagesdosis in ein bis drei Einzelrationen aufgenommen wird. Beim zweiten Präparat, das als auf den Zahnapparat bzw. das Zahnfleisch applizierbares Gel vorliegt, beträgt die Tagesdosis bevorzugtermaßen zwischen 0,2 ml und 2 ml, bevorzugt zwischen 0,5 ml und 1.5 ml.

Nach einem bevorzugten Aspekt ist das als Gel vorliegende zweite Präparat zur Aufbringung auf den Zahnapparat bzw. das Zahnfleisch bevorzugtermaßen nach der mechanischen Zahnreinigung und zur ein- oder mehrfachen Anwendung täglich vorgesehen.

Das erfindungsgemäße Zahnpflegeprodukt kann die Erhaltung von Kieferknochen und Zähnen unterstützen und/oder zur Bekämpfung bakteriell verursachter Entzündungen des Zahnfleisches oder tieferliegender Strukturen des Zahnhalteapparats beitragen, insbesondere die Bekämpfung von Parodontitis und/oder Gingivitis unterstützen. Erstes und zweites Präparat im hier vorgeschlagenen Zahnpflege-produkt-Set unterstützen sich dabei gegenseitig.

Das erste Präparat kann insbesondere die Erhaltung von Kieferknochen und Zähnen, insbesondere der Zahnstruktur selbst, des Zahnwurzelwerks und/oder des Zahnschmelzes unterstützen. Es kann darüber hinaus die normale Collagenbildung für die normale Funktion der Knochen, des Zahnfleisches und der Zähne unterstützen.

Das zweite Präparat kann dabei zur Bekämpfung bakteriell verursachter Entzündung des Zahnfleisches oder tieferliegender Strukturen des Zahnhalteapparats beitragen, insbesondere zur Bekämpfung von Parodontitis und/oder von Gingivitis beitragen.

In einem konkreten Ausführungsbeispiel wird ein Zahnpflegeprodukt-Set zur Aufbringung auf den Zahnapparat bzw. das Zahnfleisch vorgesehen, das ein erstes Präparat sowie ein zweites Präparat mit folgenden Bestandteilen umfasst:

### Erstes Präparat

| **Bestandteile auf 100 g Pulver** | | |
|---|---|---|
| Inulin | | 47 g |
| Zitronensäure (E 330) | | 20 g |
| Calciumcarbonat | | 9 g |
| Magnesiumcarbonat x 5 H₂O | | 11,5 g |
| Calciumlactat | | 5,1 g |
| Geschmacksstoffe | | 3,5 g |
| Trimagnesiumcitrat | | 0,9 g |
| Süßstoffe (Steviosid, E 960) | | 0,40 g |
| L-Ascorbinsäure (Vitamin C) | | 0,47 g |
| Zinkgluconat | | 0,32 g |
| Algenpulver | | 0,55 g |
| Betacarotin | (1 % auf Trägerstoff) | 1,24 g |
| | (=̂ 15,8 mg Betacarotin) | |
| Natriumselenit | | 0,40 mg |

### Zweites Präparat

| | |
|---|---|
| Coenzym Q10: | 2,5 Gew.-% |
| Vitamin E: | 1,7 Gew.-% (Vitamin E-Acetat) |
| Krausminze: | 3,85 Gew.-% |

### Zur Konservierung: Sorbinsäure/Sorbatmischung

Das erste, als Pulver vorliegende Präparat wird als Tagesdosis von 10 g auf zwei Einzelgaben (jeweils 5 g) mit 0,1 l bis 0,2 I Wasser angerührt und jeweils morgens und abends, beispielsweise nach der Zahnpflege oral als Drink eingenommen. Das zweite, als Gel vorliegende Präparat wird auf den Zahnapparat bzw. das Zahnfleisch bevorzugterweise nach der mechanischen Zahnreinigung und insofern mehrfach täglich vorgesehen. Unter der Annahme, dass eine mechanische Zahnreinigung durch den Anwender dreimal täglich erfolgt, wird auch eine dreimalige Aufbringung des Gels empfohlen.

Das vorliegende Zahnpflegeprodukt-Set wird vorzugsweise für verschiedene Formen von Parodontopathien eingesetzt, beispielsweise entzündliche Formen und/oder gingivo-parodontale Manifestationen systemischer Erkrankungen (Gingiva- und Parodontalveränderungen als Begleiterscheinungen zu anderen Erkrankungen) und/oder hyperplastische Formen (Formen mit vermehrter Gewebebildung) und/oder traumatogene Formen (verletzungsbedingte Formen) und/oder involutive Formen (Schwundformen).

Mit dem vorliegenden Zahnpflegeprodukt-Set kann die herkömmliche, mechanische Zahnpflege wirksam mit zwei weiteren Präparaten "von außen" sowie "von innen" wirksam unterstützt werden.

## Patentansprüche

1. Zahnpflegeprodukt-Set umfassend zwei sich in ihrer Wirkung ergänzende Präparate,
nämlich ein erstes, oral einzunehmendes Präparat,
- das als Pulver vorliegt und
- das Calcium und/oder Magnesium
- und Vitamin C
umfasst
sowie ein zweites, auf den Zahnapparat bzw. das Zahnfleisch aufzubringendes Präparat,
- das als Gel vorliegt und
- Coenzym Q10
- und Vitamin E, vorzugsweise Tocopherol, insbesondere α-Tocopherol, umfasst.

2. Zahnpflegeprodukt-Set nach Anspruch 1, **dadurch gekennzeichnet, dass** das erste Präparat als wasserlösliches Pulver vorliegt und dieses oral einzunehmende Präparat als Drink eingenommen wird.

3. Zahnpflegeprodukt-Set nach Anspruch 1 oder 2, **dadurch gekennzeichnet, dass** das erste Präparat weiterhin Inulin und/oder Zitronensäure umfasst.

4. Zahnpflegeprodukt-Set nach Anspruch 1 bis 3, **dadurch gekennzeichnet, dass** das Calcium als Calciumcarbonat und/oder als Calciumlactat vorliegt und/oder das Magnesium als Magnesiumcarbonat und/oder Magnesiumcitrat, insbesondere als Trimagnesiumcitrat vorliegt.

5. Zahnpflegeprodukt-Set nach Anspruch 1 bis 4, **dadurch gekennzeichnet, dass** bezogen auf eine Pulvermenge von 100 g
- die Menge an Calcium, das insbesondere als Calciumcarbonat und/oder als Calciumlactat vorliegt, zwischen 10 und 25 g, vorzugsweise zwischen 12 und 18 g beträgt und/oder
- die Menge an Magnesium, das insbesondere als Magnesiumcarbonat und/oder als Magnesiumcitrat vorliegt, zwischen 5 g und 20 g, vorzugsweise zwischen 8 g und 15 g beträgt und/oder
- die Menge an Vitamin C zwischen 0,2 g und 1 g, vorzugsweise zwischen 0,4 g und 0,6 g liegt und/oder
- die Menge an Inulin zwischen 30 und 50 g, vorzugsweise zwischen 40 und 45 g beträgt und/oder
- die Menge an Zitronensäure zwischen 15 g und 30 g, vorzugsweise zwischen 20 g und 28 g beträgt.

6. Zahnpflegeprodukt-Set nach einem der Ansprüche 1 bis 5, **dadurch gekennzeichnet, dass** im ersten Präparat bezogen auf 100 g Pulver Calciumcarbonat in einer Konzentration von 10 g bis 20 g, vorzugsweise von 12 bis 14 g und/oder Calciumlactat in einer Konzentration von 2 bis 5 g, vorzugsweise 2,5 bis 4 g und/oder Magnesiumcarbonat (Magnesiumcarbonat x₅H₂O) in einer Konzentration von 5 g bis 20 g, vorzugsweise von 8 g bis 13 g und/oder Magnesiumcitrat (bzw. Trimagnesiumcitrat) in einer Konzentration von 0,7 bis 2,5 g, vorzugsweise von 1 g bis 2 g, enthalten sind.

7. Zahnpflegeprodukt-Set nach einem der Ansprüche 1 bis 6, **dadurch gekennzeichnet, dass** das erste Präparat weiterhin
- Algenpulver, insbesondere Braunalgenpulver, und/oder
- Betacarotin und/oder
- Zink und/oder
- Selen und/oder
- Q10 und/oder
- Geschmacks- und/oder Aromastoffe und/oder
- Süßstoffe und/oder
- eine Steviakomponente
umfasst.

8. Zahnpflegeprodukt-Set nach Anspruch 7, **dadurch gekennzeichnet, dass** bezogen auf 100 g Pulver Algenpulver in einer Konzentration von 0,1 g bis 0,8 g und/oder Betacarotin in einer Konzentration von 5 mg bis 35 mg, vorzugsweise zwischen 10 mg und 20 mg und/oder Geschmacks- und/oder Aromastoffe in einer Konzentration von 1 g bis 4 g, insbesondere in einer Konzentration von 2 g bis 3 g und/oder Süßstoffe in Form eines Steviosids (E960) in einer Konzentration von 0,2 g bis 1,2 g, weiter vorzugsweise in einer Konzentration von 0,5 g bis 0,9 g enthalten sind.

9. Zahnpflegeprodukt-Set nach einem der Ansprüche 1 bis 8, **dadurch gekennzeichnet, dass** das Coenzym Q10, das im zweiten Präparat enthalten ist, in mycellierter Form vorliegt.

10. Zahnpflegeprodukt-Set nach einem der Ansprüche 1 bis 9, **dadurch gekennzeichnet, dass** beim zweiten Präparat pro ml Gel Coenzym Q10 in einer Konzentration von 5 mg bis 18 mg, vorzugsweise von 6 mg bis 15 mg, besonders bevorzugt von 11 mg bis 13 mg vorliegt.

11. Zahnpflegeprodukt-Set nach einem der Ansprüche 1 bis 10, **dadurch gekennzeichnet, dass** beim zweiten Präparat bezogen auf 1 mg Vitamin E in einer Konzentration von 8 mg bis 25 mg, vorzugsweise von 10 mg bis 20 mg, besonders bevorzugt von 12 mg bis 17 mg vorliegt.

12. Zahnpflegeprodukt-Set nach einem der Ansprüche 1 bis 11, **dadurch gekennzeichnet, dass** das zweite Präparat weiterhin Krausminze, vorzugsweise in einer Konzentration bezogen auf das Gel von 1 Gew.-% bis 4 Gew.-%, weiter vorzugsweise in einer Konzentration von 2 Gew.-% bis 3 Gew.-% umfasst.

13. Zahnpflegeprodukt-Set nach einem der Ansprüche 1 bis 12, **dadurch gekennzeichnet, dass** das zweite Präparat weiterhin Konservierungsstoffe, insbesondere Sorbinsäure und/oder eine Sorbatmischung umfasst.

14. Zahnpflegeprodukt-Set nach einem der Ansprüche 1 bis 13, **dadurch gekennzeichnet, dass** das in Gelform vorliegende zweite Präparat in einer Flasche mit einem Füllvolumen von 20 ml bis 40 ml, vorzugsweise von etwa 30 ml, konfektioniert wird.

15. Zahnpflegeprodukt-Set nach einem der Ansprüche 1 bis 14, **dadurch gekennzeichnet, dass** das Zahnpflegeprodukt-Set die Erhaltung von Kieferknochen und Zähnen unterstützt und/oder zur Bekämpfung bakteriell verursachter Entzündungen des Zahnfleisches oder tieferliegender Strukturen des Zahnhalteapparates beiträgt, insbesondere die Bekämpfung von Parodontitis und/oder Gingivitis unterstützt.
